# EUROPEAN PATENT SPECIFICATION

(11) **EP 3 272 314 B1**
(45) Date of publication and mention of the grant of the patent: **20.03.2019**
(21) Application number: 17275116.6
(22) Date of filing: 21.07.2017
(51) Int. Cl.: A61F 2/07, A61F 2/915

(54) **TAPERED BODY AAA GRAFT**
AAA-TRANSPLANTAT MIT KONISCHEM KÖRPER
GREFFE AAA À CORPS CONIQUE

(30) Priority: 21.07.2016 US 201662365103 P
(43) Date of publication of application: 24.01.2018
(73) Proprietor: Cook Medical Technologies LLC, Bloomington, IN 47404 (US)
(72) Inventor: HADLEY, Rick, Otterbein, IN 47970 (US); KRATZBERG, Jarin A., Lafayette, IN 47909 (US)
(74) Representative: Williams Powell

(56) References cited:
- EP-A2- 1 029 518
- WO-A1-00/74598
- US-A1- 2015 088 244

## Description

### TECHNICAL FIELD

The present invention relates to medical devices for example to endovascular devices.

### BACKGROUND

EP 1 029 518 discloses an aortic graft for treating abdominal aortic aneurysms.

US2015088244 A1 discloses first and second elongate tubular stent-graft extensions that may be percutaneously disposed into a distal end of a tubular graft body.

Endoluminal prostheses may be inserted into a body lumen such as an anatomical vessel or duct for various purposes. Prostheses may maintain or restore patency in a formerly blocked or constricted passageway or they may be used for different procedures. For example, a prosthesis may include one or more stents disposed in or about a graft, and the stents may hold the graft in an open configuration to treat an aneurysm. Additionally, stents coupled to one or both ends of a graft may extend proximally or distally away from the graft to engage a healthy portion of a vessel wall away from a diseased portion of an aneurysm to provide endovascular graft fixation.

Modular stent graft pieces can be deployed in stages to form a combined stent graft assembly. First, a central or main stent graft body can be deployed. Subsequently, secondary or attachment stent graft body can be deployed and positioned within the main stent graft body.

One of the most common complications associated with endoluminal grafting for abdominal aortic aneurisms ("AAA") is the stove piping effect that can occur in the overlap length into the proximal taper of a main stent graft body.

### SUMMARY

Insofar as the term "invention" and or "embodiment" are used in the following, and/or features are presented as being optional, this should be interpreted in such a way that the only protection sought is that of the invention as claimed.

According to an aspect of the invention, there is provided a modular stent graft system comprising:
a first stent graft having a first open end, a tapered zone tapering from a first diameter to a second diameter smaller than the first diameter, and a cylindrical zone extending from the tapered zone to a second open end;
a second stent graft configured for insertion within the first stent graft, the second stent graft comprising a first open end, a tapered zone extending from the first open end, the tapered zone of the second stent graft tapering from a third diameter to a fourth diameter smaller than the third diameter, and a cylindrical zone extending from the tapered zone of the second stent graft to a second open end of the second stent graft;
wherein the third diameter is greater than or equal to the second diameter;
wherein each tapered zone has an angle of taper and the angle of taper of the tapered zone of the second stent graft is the same as or greater than the angle of taper of the tapered zone of the first stent graft;
wherein the second stent graft comprises a first internal seal stent at the first open end of the second stent graft, the first seal stent having X top apices and a length, and a second internal seal stent partially nesting with the first internal seal stent, the second seal stent having fewer top apices than the first seal stent and a length greater than the length of the first seal stent.

The term top may for some embodiments mean closer to an inflow end. The term top may for some embodiments mean proximal.

In some embodiments, the first stent graft includes a first cylindrical zone and a second cylindrical zone, the first cylindrical zone extends for a first length from the first open end of the first stent graft to the tapered zone of the first stent graft, the tapered zone of the first stent graft extends for a second length, and the second cylindrical zone extends for a third length from the tapered zone of the first stent graft to the second open end of the first stent graft.

In some embodiments, the first cylindrical zone has the first diameter, the second cylindrical zone has the second diameter, and the cylindrical zone of the second stent graft has the fourth diameter.

In some embodiments, the diameters and angles of taper mentioned above may be unconstrained diameters and unconstrained angles of taper.

In some embodiments, apices, for example top apices, of the first seal stent interdigitate with apices, for example top apices, of the second seal stent.

In some embodiments, the cylindrical zone of the second stent graft is longer than the second cylindrical zone of the first stent graft such that when the second stent graft is disposed within the first stent graft and the tapered zones are mated, the cylindrical zone of the second stent graft extends beyond the second open end of the first stent graft.

In some embodiments, the second internal seal stent is directly adjacent to the first internal seal stent.

In some embodiments, when the second stent graft is disposed within the first stent graft, the tapered zone of the second stent graft conforms to the tapered zone of the first stent graft.

According to an aspect of the invention, there is provided a modular stent graft system comprising:
a first stent graft having a first open end, a first cylindrical zone extending from the first open end for a first length, a tapered zone extending from the cylindrical zone for a second length, the tapered zone tapering from a first diameter to a second diameter smaller than the first diameter, and a second cylindrical zone extending from the tapered zone for a third length to a second open end, wherein the third length is greater than both the first length and the second length and the first length is greater than the second length;
a second stent graft configured for insertion within the first stent graft, the second stent graft comprising a first open end, a tapered zone extending from the first open end for a first length, the tapered zone tapering from a first diameter to a second diameter smaller than the first diameter, and a cylindrical zone extending from the tapered zone to a second open end for a second length longer than the first length;
wherein the first diameter of the tapered zone of the second stent graft is greater than or equal to the first diameter of the tapered zone of the first stent graft, the second diameter of the tapered zone of the second stent graft is greater than or equal to the second diameter of the tapered zone of the first stent graft;
wherein each tapered zone has an angle of taper and the angle of taper of the tapered zone of the second stent graft is the same as the angle of taper of the tapered zone of the first stent graft;
wherein the second stent graft cylindrical zone is longer than the first stent graft second cylindrical zone such that when the second stent graft is disposed within the first stent graft and the tapered zones are mated, the second stent graft cylindrical zone extends beyond the second open end of the first stent graft;
wherein the second stent graft comprises a first internal seal stent at the first open end of the second stent graft, the first seal stent having X proximal apices and a length, and a second internal seal stent directly adjacent and partially nesting with the first internal stent, the second seal stent having ½ X top apices and a length greater than the first seal stent; and
wherein when the second stent graft is disposed within the first stent graft, the tapered zone of the second stent graft conforms precisely to the tapered zone of the first stent graft.

In some embodiments, the first stent graft has an interior and exterior and the second stent graft has an interior and exterior, wherein when the second stent graft is disposed within the first stent graft, a portion of the interior of the first stent graft directly abuts a portion of the exterior of the second stent graft in graft to graft opposition without any stents in between.

In some embodiments, the first stent graft's tapered zone has the same dimensions as the second stent graft's tapered zone.

In some embodiments, when the second stent graft is disposed within the first stent graft, the tapered zone of the second stent graft creates a seal with the tapered zone of the first stent graft.

In some embodiments, when the second stent graft comprises at least two fenestrations, and wherein, when the second stent graft is disposed within the first stent graft, the first open end of the second stent graft lies below the at least two fenestrations.

In some embodiments, the first seal stent and the second seal stent are in phase and every other top apex of the first seal stent nests with an apex of the second seal stent.

In some embodiments, the top apices of the first seal stent abut an edge of the first stent graft at the first open end.

In some embodiments, the second stent graft is a bifurcated grafting including a body portion and two leg portions extending from the body portion.

In some embodiments, the first stent graft comprises a stent disposed about the exterior of the tapered zone of the first stent graft and having top apices, and wherein, when the second stent graft is disposed in the first stent graft, the stent disposed about the exterior of the tapered zone of the first stent graft are in phase with the second seal stent.

In some embodiments, the first stent graft comprises a stent disposed about the exterior of the tapered zone of the first stent graft and has top apices and bottom apices, wherein the circumference of the stent at the top apices is greater than the circumference of the stent at the bottom apices such that the stent tapers from the top apices to the bottom apices.

In some embodiments, the second seal stent has top apices and bottom apices, wherein the circumference of the stent at the top apices is greater than the circumference of the stent at the bottom apices such that the stent tapers from the top apices to the bottom apices.

According to an aspect of the invention, there is provided a modular stent graft system comprising:
a first stent graft having a first open end, a first cylindrical zone extending from the first open end for a first length, a tapered zone extending from the cylindrical zone for a second length, the tapered zone tapering from a first diameter to a second diameter smaller than the first diameter, and a second cylindrical zone extending from the tapered zone for a third length to a second open end, wherein the third length is greater than both the first length and the second length and the first length is greater than the second length;
a second stent graft configured for insertion within the first stent graft, the second stent graft comprising a first open end, a tapered zone extending from the first open end for a first length, the tapered zone tapering from a first diameter to a second diameter smaller than the first diameter, and a cylindrical zone extending from the tapered zone to a second open end for a second length longer than the first length;
wherein the first diameter of the tapered zone of the second stent graft is the same as the first diameter of the tapered zone of the first stent graft, the second diameter of the tapered zone of the second stent graft is the same as the second diameter of the tapered zone of the first stent graft;
wherein each tapered zone has an angle of taper and the angle of taper of the tapered zone of the second stent graft is the same as the angle of taper of the tapered zone of the first stent graft;
wherein the second stent graft cylindrical zone is longer than the first stent graft second cylindrical zone such that when the second stent graft is disposed within the first stent graft and the tapered zones are mated, the second stent graft cylindrical zone extends beyond the second open end of the first stent graft, and
wherein when the second stent graft is disposed within the first stent graft, the tapered zone of the second stent graft conforms precisely to the tapered zone of the first stent graft in graft to graft apposition with no stent in between.

In some embodiments, the first stent graft has an interior and an exterior and the second stent graft has an interior and an exterior, wherein when the second stent graft is disposed within the first stent graft, a portion of the inside of the first stent graft directly abuts a portion of the outside of the second stent graft to form a fluid tight seal between the exterior of the second stent graft and the interior of the first stent graft at each of the respective tapered zones.

In some embodiments, the first stent graft's tapered zone has the same dimensions as the second stent graft's tapered zone.

In some embodiments, the first seal stent and the second seal stent are in phase and every other top apex of the first seal stent nests with an apex of the second seal stent.

In some embodiments, the top apices of the first seal stent abut an edge of the first stent graft at the first open end.

In some embodiments, the first stent graft comprises a stent disposed about the exterior of the tapered zone of the first stent graft and having top apices, and wherein, when the second stent graft is disposed in the first stent graft, the stent disposed about the exterior of the tapered zone of the first stent graft are in phase with the second seal stent.

In some embodiments, the first stent graft comprises a stent disposed about the exterior of the tapered zone of the first stent graft and has top apices and bottom apices, wherein the circumference of the stent at the top apices is greater than the circumference of the stent at the bottom apices such that the stent tapers from the top apices to the bottom apices.

According to an aspect of the disclosure there is provided a method of deploying a modular stent graft system, the method comprising:
inserting into the body a first stent graft having a first open end, a first cylindrical zone extending from the first open end for a first length, a tapered zone extending from the cylindrical zone for a second length, the tapered zone tapering from a first diameter to a second diameter smaller than the first diameter, and a second cylindrical zone extending from the tapered zone for a third length to a second open end, wherein the third length is greater than both the first length and the second length and the first length is greater than the second length;
inserting into the body a second stent graft within the first stent graft, the second stent graft comprising a first open end, a tapered zone extending from the first open end for a first length, the tapered zone tapering from a first diameter to a second diameter smaller than the first diameter, and a cylindrical zone extending from the tapered zone to a second open end for a second length longer than the first length; wherein the first diameter of the tapered zone of the second stent graft is the same as the first diameter of the tapered zone of the first stent graft, the second diameter of the tapered zone of the second stent graft is the same as the second diameter of the tapered zone of the first stent graft, wherein each tapered zone has an angle of taper and the angle of taper of the tapered zone of the second stent graft is the same as the angle of taper of the tapered zone of the first stent graft, wherein the second stent graft cylindrical zone is longer than the first stent graft second cylindrical zone such that when the second stent graft is disposed within the first stent graft and the tapered zones are mated, the second stent graft cylindrical zone extends beyond the second open end of the first stent graft, wherein when the second stent graft is disposed within the first stent graft, the tapered zone of the second stent graft conforms precisely to the tapered zone of the first stent graft to create a fluid tight seal between graft material between the tapered zones without any stent in between.

In some examples, the second stent graft's tapered zone is deployed within the first stent graft's tapered zone.

Some embodiments provide an apparatus for deployment of a modular stent-graft system. In one embodiment, a modular system comprises a first stent graft having a tapered zone, a second stent graft configured for insertion within the first stent graft, the second stent graft comprising a tapered zone, wherein each tapered zone has an angle of taper and the angle of taper of the tapered zone of the second stent graft is substantially the same as the angle of taper of the tapered zone of the first stent graft, and wherein when the second stent graft is disposed within the first stent graft, the tapered zone of the second stent graft conforms precisely to the tapered zone of the first stent graft.

According to an aspect of the present disclosure, there is provided a modular stent graft system comprising a first stent graft having a first open end, a first cylindrical zone extending from the first open end for a first length, a tapered zone extending from the cylindrical zone for a second length, the tapered zone tapering from a first diameter to a second diameter smaller than the first diameter, and a second cylindrical zone extending from the tapered zone for a third length to a second open end, wherein the third length is greater than both the first length and the second length and the first length is greater than the second length; a second stent graft configured for insertion within the first stent graft, the second stent graft comprising a first open end, a tapered zone extending from the first open end for a first length, the tapered zone tapering from a first diameter to a second diameter smaller than the first diameter, and a cylindrical zone extending from the tapered zone to a second open end for a second length longer than the first length; wherein the first diameter of the tapered zone of the second stent graft is substantially the same as the first diameter of the tapered zone of the first stent graft, the second diameter of the tapered zone of the second stent graft is substantially the same as the second diameter of the tapered zone of the first stent graft, wherein each tapered zone has an angle of taper and the angle of taper of the tapered zone of the second stent graft is substantially the same as the angle of taper of the tapered zone of the first stent graft, wherein the second stent graft cylindrical zone is longer than the first stent graft second cylindrical zone such that when the second stent graft is disposed within the first stent graft and the tapered zones are mated, the second stent graft cylindrical zone extends beyond the second open end of the first stent graft, wherein the second stent graft comprises a first internal seal stent at the first open end of the second stent graft, the first seal stent having X proximal apices and a length, and a second internal seal stent directly adjacent and partially nesting with the first internal stent, the second seal stent having ½ X top apices and a length greater than the first seal stent; and wherein when the second stent graft is disposed within the first stent graft, the tapered zone of the second stent graft conforms precisely to the tapered zone of the first stent graft.

According to an aspect of the present disclosure, there is provided a modular stent graft system comprising: a first stent graft having a first open end, a first cylindrical zone extending from the first open end for a first length, a tapered zone extending from the cylindrical zone for a second length, the tapered zone tapering from a first diameter to a second diameter smaller than the first diameter, and a second cylindrical zone extending from the tapered zone for a third length to a second open end, wherein the third length is greater than both the first length and the second length and the first length is greater than the second length; a second stent graft configured for insertion within the first stent graft, the second stent graft comprising a first open end, a tapered zone extending from the first open end for a first length, the tapered zone tapering from a first diameter to a second diameter smaller than the first diameter, and a cylindrical zone extending from the tapered zone to a second open end for a second length longer than the first length; wherein the first diameter of the tapered zone of the second stent graft is substantially the same as the first diameter of the tapered zone of the first stent graft, the second diameter of the tapered zone of the second stent graft is substantially the same as the second diameter of the tapered zone of the first stent graft; wherein each tapered zone has an angle of taper and the angle of taper of the tapered zone of the second stent graft is substantially the same as the angle of taper of the tapered zone of the first stent graft; and wherein the second stent graft cylindrical zone is longer than the first stent graft second cylindrical zone such that when the second stent graft is disposed within the first stent graft and the tapered zones are mated, the second stent graft cylindrical zone extends beyond the second open end of the first stent graft, and wherein when the second stent graft is disposed within the first stent graft, the tapered zone of the second stent graft conforms precisely to the tapered zone of the first stent graft.

According to an aspect of the present disclosure, there is disclosed a method of deploying a modular stent graft system, the method comprising: inserting into the body a first stent graft having a first open end, a first cylindrical zone extending from the first open end for a first length, a tapered zone extending from the cylindrical zone for a second length, the tapered zone tapering from a first diameter to a second diameter smaller than the first diameter, and a second cylindrical zone extending from the tapered zone for a third length to a second open end, wherein the third length is greater than both the first length and the second length and the first length is greater than the second length; inserting into the body a second stent graft within the first stent graft, the second stent graft comprising a first open end, a tapered zone extending from the first open end for a first length, the tapered zone tapering from a first diameter to a second diameter smaller than the first diameter, and a cylindrical zone extending from the tapered zone to a second open end for a second length longer than the first length; wherein the first diameter of the tapered zone of the second stent graft is substantially the same as the first diameter of the tapered zone of the first stent graft, the second diameter of the tapered zone of the second stent graft is substantially the same as the second diameter of the tapered zone of the first stent graft, wherein each tapered zone has an angle of taper and the angle of taper of the tapered zone of the second stent graft is substantially the same as the angle of taper of the tapered zone of the first stent graft, wherein the second stent graft cylindrical zone is longer than the first stent graft second cylindrical zone such that when the second stent graft is disposed within the first stent graft and the tapered zones are mated, the second stent graft cylindrical zone extends beyond the second open end of the first stent graft, wherein when the second stent graft is disposed within the first stent graft, the tapered zone of the second stent graft conforms precisely to the tapered zone of the first stent graft.

Preferred embodiments can provide a stent graft assembly that reduces the stove piping effect and reduces the chance of turbulent flow of blood and possible thrombotic buildup through the main stent graft body.

### BRIEF DESCRIPTIONS OF THE DRAWINGS

Embodiments of the present invention are described below, by way of example only with reference to the accompanying drawings, in which:
Figure 1 illustrates one example of a main stent graft body or device.
Figure 2 illustrates one example of an attachment stent graft having a flared proximal end.
Figure 3 illustrates the proximal end of the attachment stent graft shown in Figure 2.
Figure 4 illustrates one example of a main stent graft body coupled to an attachment stent graft at a tapered mating area.
Figure 5 illustrates one example of a main stent graft body coupled to an attachment stent graft at a distal mating area.
Figure 6 illustrates a detailed view of the tapered mating area shown in Figure 4.
Figure 7 illustrates a perspective view of the assembly of Figure 6.
Figure 8 illustrates a detailed view of the distal mating area shown in Figure 5.
Figure 9 illustrates a perspective view of the assembly of Figure 8.
Figure 10 illustrates the flow of blood through a known stent graft assembly.
Figure 11 illustrates the flow of blood through a stent graft assembly.
Figure 12 illustrates the flow of blood through a known stent graft assembly.
Figure 13 illustrates the flow of blood through a stent graft assembly.

### DETAILED DESCRIPTION

In the present application, the term "proximal" when referring to a delivery device refers to a direction that is farthest away from the operator using a delivery device, while the term "distal" refers to a direction that is generally closest to the operator using the delivery device. The proximal and distal ends of a delivery device can also be referred to as the introduction end of the delivery device and the operator end of the delivery device. The operator end of the delivery device is that portion of the device that is intended to remain outside of a patient during a procedure. When referring to the prosthesis itself relative to the delivery device, the proximal end of the prosthesis is that part of the prosthesis nearest the delivery end of the delivery device and the distal end of the prosthesis is that end that is closest to the operator end of the delivery device. When referring to the prosthesis relative to placement in the human body, the ends of the various devices and parts of devices may be referred to as the inflow end (that end that receives fluid first, and the outflow end (that end from which the fluid exits).

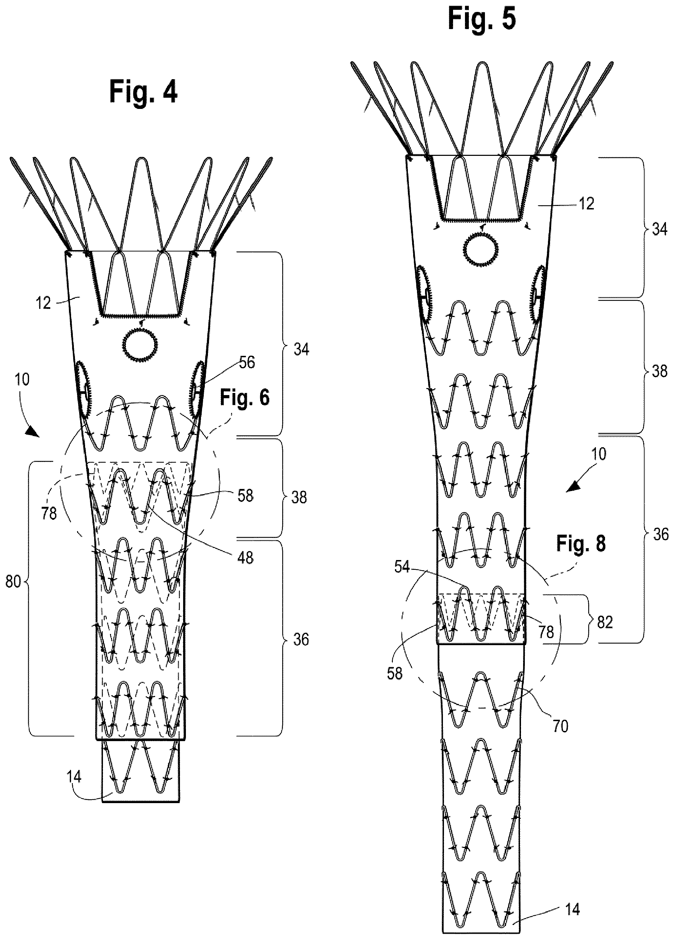

The stent graft assembly 10 of the present invention (shown for example in Figs. 4-5) may include a main or first stent graft body or device 12 and a second graft body or attachment stent graft 14. In one example, the main stent graft body 12 may be a Cook Medical Zenith Pivoting Branch Graft showing pivoting fenestrations as disclosed in US Patent Nos. 8702786, 8795349, 9351822, 8870939 and US Patent Publication No. 2014/0277335. In one example, the main stent graft body 12 or attachment stent graft 14 may be a fenestrated graft such as those shown in US Patent Nos. 9072621, 9072621, 8523934, 9060887, 8172895, 7833259, 7413573, 9149382, and US Publication Nos. 2015-0112420, 2016-0022411. In one example, the attachment stent graft 14 may be a Cook Medical Zenith AAA Stent Graft.

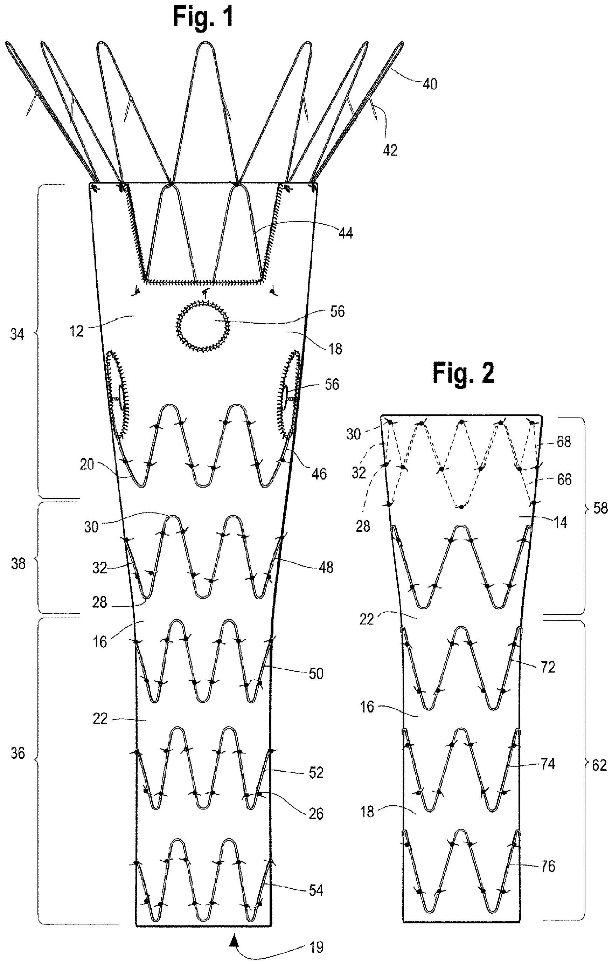

The main stent graft body 12 and attachment stent graft 14 will now be described in greater detail. Figure 1 illustrates one example of a main stent graft body or device 12. Figure 2 illustrates one example of an attachment stent graft 14 having a flared proximal end. In this embodiment, the main stent graft body 12 and attachment stent graft 14 may comprise a tubular body 16 of a biocompatible graft material 18 with a lumen 19 running therethrough.

The main stent graft body 12 and attachment stent graft 14 may be supported with one or more stents 20 that are secured to and along the graft material 18 either along the outer surface 22 or inner surface 24 of the graft material 18 such as by sutures 26. In one example, stent 20 may be a Z-stent. For example, stent 20 may have a distal end with a series of distal apices 28 and a proximal end with a series of proximal apices 30. Stent 20 may also have one or more elongate struts 32 connecting the distal apices 28 to the proximal apices 30.

Suitable stents 20 for use in connection with the main stent graft body 12 or the attachment stent graft 14 described herein may be self-expanding or mechanically-expandable stents or both, and may be deployed according to conventional methodology. A self-expanding stent may be manufactured from a shape-memory alloy, such as nickel titanium alloy (Nitinol). If the stent comprises a self-expanding material such as Nitinol, the stent may be heat-set into the desired expanded state whereby the stent can assume a relaxed radially expanded configuration. The stent may be made from other metals and alloys that allow the stent to return to its original expanded configuration upon deployment, such as, for example, stainless steel, cobalt-chrome alloys, amorphous metals, and/or non-metallic materials as would be recognized by one of skill in the art. Additionally or alternatively, the main stent graft body 12 and the attachment stent graft 14 may be mechanically expanded, such as through the use of an expandable balloon placed within the lumen 19 of the stent graft and then radially outwardly expanded.

The main stent graft body 12 may have a proximal end portion 34 or zone, which may in some embodiments be cylindrical, and a distal cylindrical end portion or zone 36, with a tapered transition portion or zone 38 that interconnects the proximal end portion 34 and the distal end portion 36. The proximal end portion may extend from a first open end to the tapered portion and the distal end portion may extend from the tapered portion to a second open end. The distal end portion 36 may have a constant diameter and the proximal end portion 34 may have a selected larger diameter. The tapered portion 38 may therefore taper from the diameter of the proximal end portion 34 to the diameter of the distal end portion 36. The proximal end portion may have a first length, the tapered portion may have a second length, and the distal end portion may have a third length. The third length may be greater than both the first and second lengths and the first length may be greater than the second length. An attachment stent 40 may be secured to the proximal end portion 34, with the attachment stent 40 extending proximally from the graft material 18. The attachment stent 40 may have one or more barbs 42 configured to secure the main stent graft body 12 to a vessel wall.

The main stent graft body 12 may have several additional stents 44, 46, 48, 50, 52 and 54 adjacent to the attachment stent 40. In one example, stent 44 may be secured to the inner surface 24 of the graft material 18 in the proximal end portion 34 of the main stent graft body 12. Stents 46, 48, 50, 52, and 54 may be secured about the outer surface 22 of the graft material 18 along the length thereof. In one example, stents 46, 48, 50, 52, and 54 are located distal to the attachment stent 40 and stent 44.

The proximal end portion 34 may contain a plurality of radiopaque markers (not shown) such as gold marker members for facilitating fluoroscopic visualization of the proximal end of the graft material 18. For example, radiopaque markers may be used to place the main body stent graft 12 distal to the renal arteries (not shown).

As shown in Figure 1, one or more fenestrations 56 may be placed on the biocompatible graft material 18. These fenestrations 56 may substantially align with the expected position of a branch vessel in the patient. There is, however, some variation both circumferentially and longitudinally in the position of the vessels.

Figure 2 illustrates one example of an attachment stent graft 14 having a flared proximal end 58. The attachment stent graft 14 may be configured to pair with the main stent graft body 12. In one example, the main stent graft body 12 is configured to receive the attachment stent graft 14 as described in greater detail below.

The attachment stent graft 14 shown in Figure 2 may have a flared or tapered proximal end portion or zone 58 and a cylindrical distal end portion or zone 62. The distal end portion 62 may have a constant diameter and a proximal open end of the flared proximal end portion 58 may have a selected larger diameter. The flared portion 58 may therefore taper from the diameter of the proximal open end to the diameter of the distal end portion 62. The distal end portion 62 may be a unibody tube (as shown), or it may be a bifurcated stent graft having a body portion and two or more legs. In one example, the bifurcated stent graft comprises a shorter leg and a longer leg. The distal end portion 62 may extend for a length longer than a length of the flared portion 58 and may extend from the flared portion 58 and have an open distal end. The distal end portion 62 may be longer than the distal end portion 36 of the main stent graft body.

The attachment stent graft 14 may have several stents 66, 68, 70, 72, 74, and 76 which may be secured to the graft material 18 along the length of the attachment stent graft 14. In one example, a first stent 66 and a second stent 68 may be secured to the inner surface 24 of the graft material 18 near the flared proximal end portion 58. In one example, additional stents 70, 72, 74, and 76 may be secured about the outer surface 22 of the graft material 18.

Importantly, the proximal end of the attachment stent graft 14 may have a flared proximal end portion 58. In one example, the flared portion 58 may complement or conform to the transition portion 38 on the interior of the main stent graft body 12. For example, as seen comparing Figures 1 and 2 side by side, the flared proximal end 58 of the attachment stent graft 14 and the transition portion 38 of the main stent graft body 12 are similar in shape. The complementary flared proximal end 58 of the attachment stent graft 14 and the transition portion 38 of the main stent graft body 12 may allow a better mating area between the main stent graft body 12 and the attachment stent graft 14 (as described below and shown in Figures 4-9).

In some embodiments, in order to enhance mating, the diameter of the proximal end of the flared portion of the attachment stent graft may be greater than or equal to the diameter of the distal end portion 36 of the main stent graft body. In addition, the diameter of the distal end portion 62 of the attachment stent graft may be greater than or equal to the diameter of the distal end portion 36 of the main stent graft body. In addition, an angle of taper of the flared portion 58 of the attachment stent graft may be the same as or greater than, preferably the same as, an angle of taper of the tapered portion of the main stent graft body.

In some embodiments, the diameter of the proximal end of the flared portion 58 of the attachment stent graft may be greater than or equal to the diameter of the proximal end portion 34 of the main stent graft body.

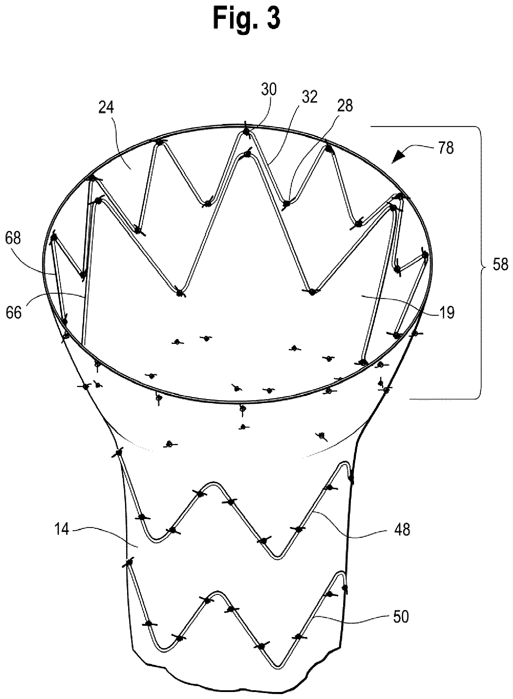

Figure 3 illustrates the proximal end of the attachment stent graft shown in Figure 2. In one example, the flared proximal end portion 58 of the attachment stent graft 14 may have a stacked stent arrangement 78. The stacked stent arrangement 78 may be located in the flared proximal end 58 of the attachment stent graft 14. The stacked stent arrangement 78 may allow for a tighter seal when the attachment stent graft 14 is placed into a lumen 19 in the main stent graft body 12.

In one example of a stacked stent arrangement 78, a first stent 66, which may in some embodiments be considered a first internal seal stent, and a second stent 68, which may in some embodiments be considered a second internal seal stent, are mounted on an interior surface 24 of the graft material 18 in the attachment stent graft 14. The first stent 66 and second stent 68 may be formed as a wire ring that has proximal and distal generally curved apex portions (apices) 30, 28 separated from each other by intermediate generally straight portions called struts 32.

In some embodiments, the second stent 68 has X proximal apices and the first stent 66 has ½X proximal apices. In some embodiments, the second stent 68 may be at the proximal open end of the flared portion 58 and proximal apices of the second stent 68 may abut an edge of the open end of the flared portion 58.

In some embodiments, a circumference of the first stent 66 at proximal apices thereof is greater than a circumference of the first stent 66 at distal apices thereof such that the first stent 66 tapers from the proximal apices thereof to the distal apices thereof.

First stent 66 and second stent 68 may not have the same proximal to distal length. For example, as illustrated in Figure 3 the second stent 68 has a shorter proximal-distal length than the first stent 66. In another embodiment (not shown), the first stent 66 and the second stent 68 may have the same proximal to distal length.

In the embodiment of Figure 3 the first stent 66 and second stent 68 may overlap axially such that apices of the second stent 68 nest within apices of the first stent 66. For example, every other proximal apex of the second stent 68 nests with a proximal apex of the first stent 66. In another embodiment the first stent 66 and second stent 68 do not overlap or nest. For example, the first stent 66 and second stent 68 do not overlap axially, but are very close together. In one example, the first stent 66 is located slightly distal to the second stent 68.

In one embodiment (not shown), each of the proximal apices 30 of the first stent 66 may be circumferentially offset from each of the proximal apices 30 of the second stent 68. In another example, each of the distal apices 30 of the first stent 66 may be circumferentially offset from each of the distal apices 30 of the second stent 68. In another example, the first stent 66 and the second stent 68 comprise identical geometries. The two stents 66 and 68 may be arranged in an out-of-phase alignment or may be in-phase alignment. The first stent 66 and second stent 68 may have any configuration and geometry as disclosed in US Patent No. 8,728,145.

The attachment stent graft 14 and the main stent graft body 12 may be coupled or connected. Figures 4 and 5 illustrate two examples of a main stent graft body 12 coupled to an attachment stent graft 14.

In one example, the attachment stent graft 14 may have a reduced diameter delivery configuration (not shown) and a deployment configuration. When in the delivery configuration, the attachment stent graft 14 can be deployed into a lumen 19 in the main stent graft body 12.

The attachment stent graft 14 can be positioned and deployed to any location along the length of the main stent graft body 12. In this way, the stent graft assembly 10 may be any suitable customizable length. For example, if the attachment stent graft 14 is placed in a more proximal location of the main stent graft 12, the total length of the stent graft assembly 10 may be shorter. If the attachment stent graft 14 is placed in a more distal location on the main stent graft 12, the total length of the stent graft assembly 10 may be longer.

When in a deployed state, the attachment stent graft 14 may expand and conform to the shape of the interior of the main stent graft body 12.

Figure 4 illustrates one example of a main stent graft body 12 coupled to an attachment stent graft 14 at a tapered mating area 80. As illustrated in Figure 4, the flared proximal end 58 of the attachment stent graft 14 may be coupled to the main stent graft body 12 at a tapered mating area 80. The tapered mating area 80 may be located in the area just distal of fenestrations 56. A portion of the tapered mating area 80 may be in the tapered transition portion 38 that interconnects the proximal end portion 34 and the distal end portion 36 of the main stent graft body 12. The flared proximal end 58 of the attachment stent graft 14 may conform to the shape of the tapered transition portion 38 of the main stent graft body 12.

The radial force of the stacked stent arrangement 78 may hold the attachment stent graft 14 tightly against the interior of the main stent graft body 12. In this way, there is a tight seal between the attachment stent graft 14 and the main stent graft body 12. As described in more detail below, this tight seal can prevent turbulent vascular flow through the lumen 19 of the stent graft assembly 10.

Figure 5 illustrates one example of a main stent graft body 12 coupled to an attachment stent graft 14 at a distal mating area 82. As illustrated in Figure 5, the flared proximal end 58 of the attachment stent graft 14 may be coupled to the main stent graft body 12 at a distal mating area 82. The distal mating area 82 may be in the distal end portion 36 of the main stent graft body.

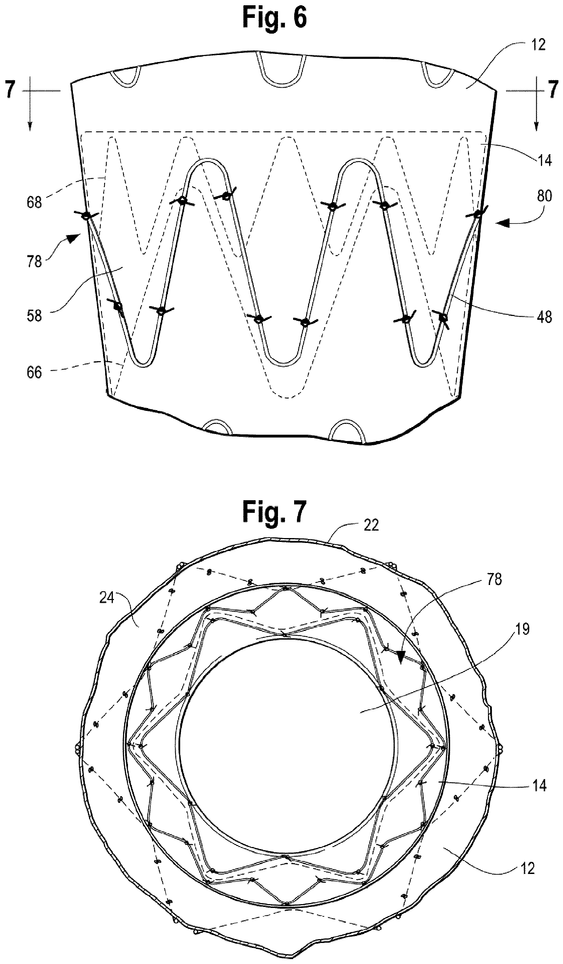

Figure 6 illustrates a close up of the tapered mating area 80 shown in Figure 4. The attachment stent graft 14 may be disposed in the lumen 19 of the main stent graft body 12. In this example, the flared proximal end 58 of the attachment stent graft 14 is disposed in the main stent graft body 12 in the tapered mating area 80. The stacked stent arrangement 78 mounted on the interior graft 24 in the flared proximal end 58 of the attachment stent graft 14 may be at the same longitudinal level as stent 48 on the main stent graft body 12. In some embodiments, the stent 48 is an exterior stent disposed about the exterior of the tapered portion 38 and, when the attachment stent graft is disposed in the main stent graft body, the stent 48 is in phase with the first stent 66. In some embodiments, a circumference of the stent 48 at proximal apices thereof is greater than a circumference of the stent 48 at distal apices thereof such that the stent 48 tapers from the proximal apices thereof to the distal apices thereof.

Figure 7 illustrates a perspective view or end view of the assembly of Figure 6. The main stent graft body 12 may have a lumen 19 extending therethrough. The attachment stent graft 14 may be disposed in the lumen 19 of the main stent graft body 12. A stacked stent arrangement 78 may be disposed on the flared proximal end 58 of the attachment stent graft 14.

As shown in Figure 7, the shape of the flared proximal end 58 of the attachment stent graft 14 and the tapered transition portion 38 of the main stent graft body 12 are complimentary such that a tight seal can be created between the main stent graft body 12 and the attachment stent graft 14. As will be described in greater detail below, a tight seal can prevent turbulent vascular flow through the lumen 19 of the assembly 10.

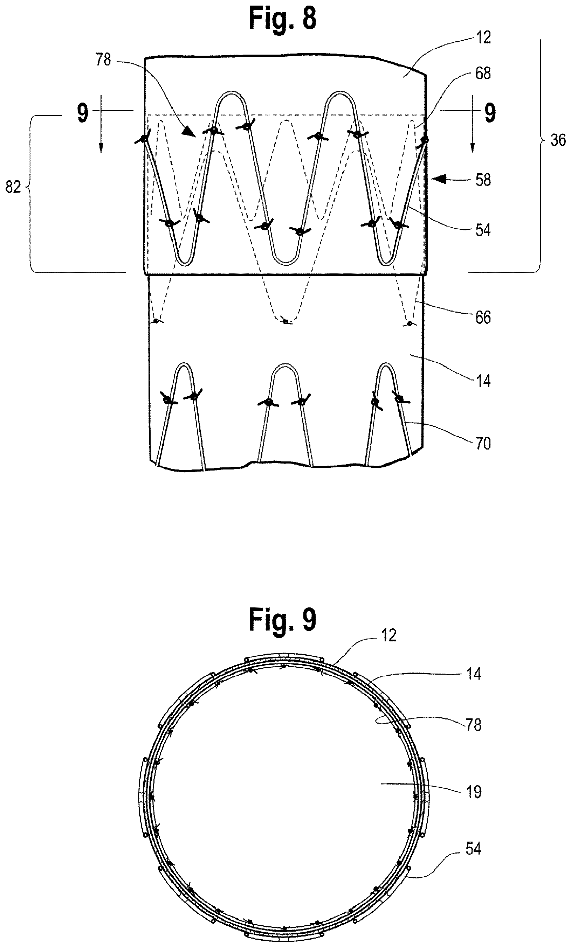

Figure 8 illustrates a detailed view of the distal mating area 82 illustrated in Figure 5. The attachment stent graft 14 may be disposed in the lumen 19 of the main stent graft body 12. In this example, the flared proximal end 58 of the attachment stent graft 14 is disposed in the main stent graft body 12 in the distal mating area 82.

As shown in Figure 8, a stent 54 on the distal portion 36 of the main stent graft body 12 may be located on the outer surface of the main stent graft body 12. The attachment stent graft 14 may have a stacked stent arrangement 78 mounted on an interior surface 24 of the flared proximal end 58 of the attachment stent graft 14. The stacked stent arrangement 78 may be at the same longitudinal level as a stent 54 on the distal portion 36 of the main stent graft body 12.

Figure 9 illustrates a perspective view or end view of the assembly of Figure 8. The main stent graft body 12 may have a lumen 19 extending therethrough. The attachment stent graft 14 may be disposed in the lumen 19 of the main stent graft body 12. A stacked stent arrangement 78 may be disposed on the flared proximal end 58 of the attachment stent graft 14. In this example, the attachment stent graft 14 is disposed at the distal end portion 36 of the main stent graft body 12.

As shown in Figure 9, a tight seal can be created between the main stent graft body 12 and the attachment stent graft 14. As will be described in greater detail below, a tight seal can prevent turbulent vascular flow through the lumen 19 of the stent graft assembly 10.

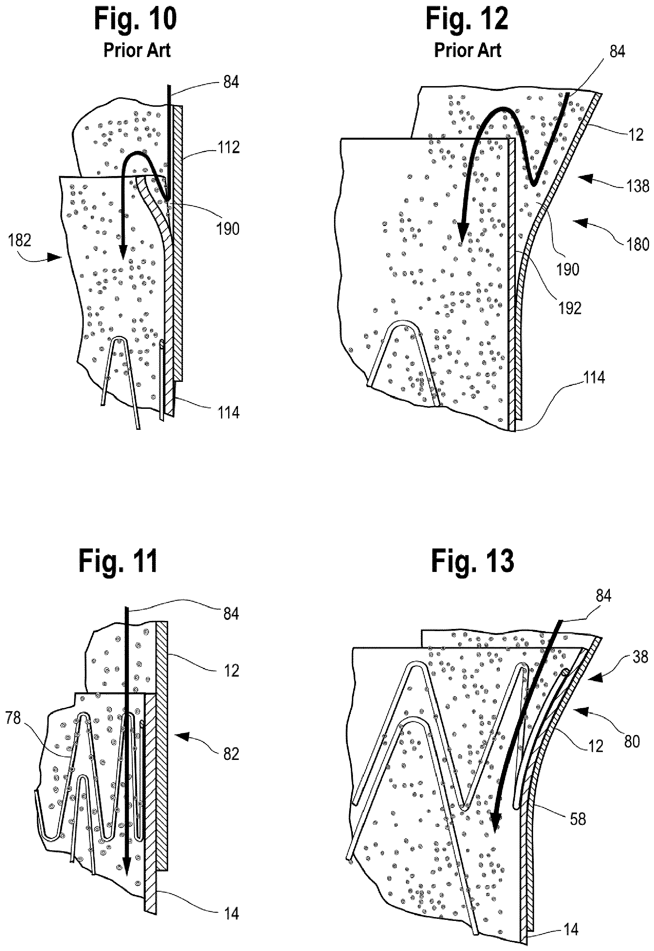

Figure 10 illustrates the flow of blood through a known stent graft assembly 110 where a main stent graft body 112 is coupled to a conventional attachment stent graft 114 at a distal mating area 182. As shown in Figure 10, blood flow 84 can travel in a proximal 86 to distal 88 direction through the lumen 119 of the main stent graft body 112. A conventional attachment graft 114 may be disposed in the lumen 119 of the main stent graft body 112. The seal between the conventional attachment graft 114 and the main stent graft body 112 may not be very tight and stove piping may occur. When stove piping occurs, the blood flow 84 may be turbulent at the transition between the main stent graft body 112 and the conventional attachment graft 114. As shown in Figure 10, the blood flow may not take a straight path through the lumen of the stent graft assembly 10 but rather may get caught in a gap 190 in a distal mating area 182.

Figure 11 illustrates the flow of blood 84 through a stent graft assembly 10 of the present disclosure where a main stent graft body 12 is coupled to an attachment stent graft 14 at a distal mating area 82 wherein the proximal end of the attachment stent graft has a stacked stent arrangement. As shown in Figure 11, a tight seal created by the stacked stent arrangement 78 in the attachment stent graft 14 can allow for blood to flow in a relatively straight path in a proximal 86 to distal 88 direction. The tighter seal can reduce the stove piping effect that can be seen with conventional devices. It can also reduce the risk of turbulent blood flow and also reduces the risk for thrombolytic build-up at a seal area 82. The tighter seal created by the stacked stent arrangement 78 can also allow a constant seal. The stacked stent arrangement 78 at the proximal end of the attachment stent graft 14 can be flexible and adjustable and allow for deep connection delivery of grafts.

Figure 12 illustrates the flow of blood through a known stent graft assembly 10 where a main stent graft body is coupled to an attachment stent graft at a tapered mating area. As shown in Figure 12, a conventional attachment stent graft 14 may have a straight proximal end 192 (as opposed to the tapered proximal end shown in this disclosure at Figures 2-4). As a result, a gap 190 may occur between the tapered transition area 138 on the main stent graft body 12 and the proximal end 192 of the conventional attachment stent graft 14. Blood flowing through the assembly 10 may not take a straight path through the lumen of the stent graft assembly 10 but rather the blood may get caught in a gap 190 in a seal area 180. This gap can create a turbulent blood flow.

Figure 13 illustrates the flow of blood through a stent graft assembly 10 where a main stent graft body 12 is coupled to an attachment stent graft 14 at a tapered mating area 80 wherein the flared proximal end 58 of the attachment stent graft 14 is tapered and has a stacked stent arrangement. In this arrangement, the tapered proximal portion 58 of the attachment stent graft 14 may create a tight seal with the tapered transition portion 38 of the main stent graft body 12.

As shown in Figure 13, the tighter seal created by the stacked stent arrangement 78 and the tapered proximal portion 58 of the attachment stent graft 14 can allow for blood to flow in a relatively straight path in a proximal 86 to distal 88 direction. The tighter seal can reduce the stove piping effect that can be seen with conventional devices. It can also reduce the risk of turbulent blood flow and also reduces the risk for thrombolytic build-up at a seal area 80. The tighter seal created by the stacked stent arrangement 78 and tapered transition portion 58 can also allow a constant seal. The stacked stent arrangement 78 and the tapered proximal portion 58 of the attachment stent graft 14 can be flexible and adjustable and allow for deep connection delivery of grafts.

While various embodiments of the invention have been described, the invention is not to be restricted except in light of the attached claims and their equivalents.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another, as long as the resulting embodiment falls within the scope of the appended claims.

## Claims

1. A modular stent graft system comprising:
a first stent graft (12) having a first open end, a tapered zone (38) tapering from a first diameter to a second diameter smaller than the first diameter, and a cylindrical zone (36) having the second diameter extending from the tapered zone to a second open end;
a second stent graft (14) configured for insertion within the first stent graft (12), the second stent graft (14) comprising a first open end, a tapered zone (58) extending from the first open end of the second stent graft (14), the tapered zone (58) of the second stent graft tapering from a third diameter to a fourth diameter smaller than the third diameter, and a cylindrical zone extending from the tapered zone of the second stent graft (14) to a second open end of the second stent graft (14);
wherein the third diameter is greater than or equal to the second diameter;
wherein each tapered zone has an angle of taper and the unconstrained angle of taper of the tapered zone of the second stent graft (14) is the same as or greater than the angle of taper of the tapered zone of the first stent graft (12);
wherein the second stent graft (14) comprises a first internal seal stent (68) at the first open end of the second stent graft (14), the first seal stent having X top apices and a length, and a second internal seal stent (66) partially nesting with the first internal seal stent, the second seal stent having fewer top apices than the first seal stent and a length greater than the length of the first seal stent.

2. The modular stent graft system of claim 1, wherein the fourth diameter is greater than or equal to the second diameter.

3. The modular stent graft system of claim 1 or 2, wherein the second internal seal stent has ½ X top apices.

4. The modular stent graft system of any preceding claim, wherein the cylindrical zone of the second stent graft (14) is longer than the cylindrical zone of the first stent graft (12).

5. The modular stent graft system of any preceding claim, wherein the first stent graft (12) includes a first cylindrical zone and a second cylindrical zone (36); wherein the first cylindrical zone extends for a first length from the first open end of the first stent graft (12) to the tapered zone (38) of the first stent graft (12), the tapered zone (38) of the first stent graft (12) extends for a second length, and the second cylindrical zone (36) extends for a third length from the tapered zone (38) of the first stent graft (12) to the second open end of the first stent graft (12); wherein the third length is greater than both the first length and the second length and the first length is greater than the second length.

6. The modular stent graft system of any preceding claim, wherein the cylindrical zone of the second stent graft (14) is longer than the tapered zone of the second stent graft (14).

7. The modular stent graft system of any preceding claim, wherein the first stent graft (12) has an interior and exterior and the second stent graft (14) has an interior and exterior, wherein when the second stent graft (14) is disposed within the first stent graft, a portion of the interior of the first stent graft directly abuts a portion of the exterior of the second stent graft (14) in graft to graft opposition without any stents in between.

8. The modular stent graft system any preceding claim, wherein when the second stent graft (14) is disposed within the first stent graft (12), the tapered zone of the second stent graft (14) creates a seal with the tapered zone of the first stent graft (12).

9. The modular stent graft system of any preceding claim, wherein the first stent graft (12) comprises at least two fenestrations, and wherein, when the second stent graft (14) is disposed within the first stent graft (12), the first open end of the second stent graft lies below the at least two fenestrations.

10. The modular stent graft system of any preceding claim, wherein the first seal stent and the second seal stent are in phase and every other top apex of the first seal stent nests with an apex of the second seal stent.

11. The modular stent graft system of any preceding claim, wherein the top apices of the first seal stent abut an edge of the second stent graft at the first open end thereof.

12. The modular stent graft system of any preceding claim, wherein the second stent graft (14) is a bifurcated grafting including a body portion and two leg portions extending from the body portion.

13. The modular stent graft system of any preceding claim, wherein the first stent graft (12) comprises a stent disposed about an exterior of the tapered zone of the first stent graft (12), and wherein, when the second stent graft (14)is disposed in the first stent graft, the stent disposed about the exterior of the tapered zone of the first stent graft is in phase with the second seal stent.

14. The modular stent graft of any preceding claim, wherein the first stent graft (12) comprises an exterior stent disposed about an exterior of the tapered zone of the first stent graft (12), the said exterior stent having top apices and bottom apices, wherein a circumference of the said exterior stent at the top apices thereof is greater than a circumference of the said exterior stent at the bottom apices thereof such that the said exterior stent tapers from the top apices thereof to the bottom apices thereof.

15. The modular stent graft of claim 14, wherein the second seal stent has top apices and bottom apices, wherein a circumference of the second seal stent at the top apices thereof is greater than a circumference of the second seal stent at the bottom apices thereof such that the second seal stent tapers from the top apices thereof to the bottom apices thereof.

## Patentansprüche

1. Modulares Stentgraftsystem, umfassend:
einen ersten Stentgraft (12) mit einem ersten offenen Ende, einem sich verjüngenden Bereich (38), der sich von einem ersten Durchmesser zu einem zweiten Durchmesser verjüngt, der kleiner als der erste Durchmesser ist, und einem zylindrischen Bereich (36) mit dem zweiten Durchmesser, der sich vom sich verjüngenden Bereich zu einem zweiten offenen Ende erstreckt,
einen zweiten Stentgraft (14), der zum Einsetzen in den ersten Stentgraft (12) ausgestaltet ist,
wobei der zweite Stentgraft (14) ein erstes offenes Ende, einen sich verjüngenden Bereich (58), der sich von dem ersten offenen Ende des zweiten Stentgrafts (14) erstreckt, wobei sich der sich verjüngende Bereich (58) des zweiten Stentgrafts von einem dritten Durchmesser zu einem vierten Durchmesser verjüngt, der kleiner als der dritte Durchmesser ist, und einen zylindrischen Bereich umfasst, der sich von dem sich verjüngenden Bereich des zweiten Stentgrafts (14) zu einem zweiten offenen Ende des zweiten Stentgrafts (14) erstreckt,
wobei der dritte Durchmesser größer als der oder gleich dem zweiten Durchmesser ist,
wobei jeder sich verjüngende Bereich einen Kegelwinkel hat und der nicht eingeschränkte Kegelwinkel des sich verjüngenden Bereichs des zweiten Stentgrafts (14) derselbe wie der Kegelwinkel des sich verjüngenden Bereichs des ersten Stentgrafts (12) oder größer als dieser ist,
wobei der zweite Stentgraft (14) einen ersten internen Dichtungsstent (68) am ersten offenen Ende des zweiten Stentgrafts (14) umfasst, wobei der erste Dichtungsstent X obere Scheitel und eine Länge hat und der zweite interne Dichtungsstent (66) teilweise in den ersten internen Dichtungsstent eingeschachtelt ist, wobei der zweite Dichtungsstent weniger obere Scheitel als der erste Dichtungsstent und eine Länge hat, die größer als die Länge des ersten Dichtungsstents ist.

2. Modulares Stentgraftsystem nach Anspruch 1, wobei der vierte Durchmesser größer als der oder gleich dem zweiten Durchmesser ist.

3. Modulares Stentgraftsystem nach Anspruch 1 oder 2, wobei der zweite interne Dichtungsstent ½ X obere Scheitel hat.

4. Modulares Stentgraftsystem nach einem der vorhergehenden Ansprüche, wobei der zylindrische Bereich des zweiten Stentgrafts (14) länger als der zylindrische Bereich des ersten Stentgrafts (12) ist.

5. Modulares Stentgraftsystem nach einem der vorhergehenden Ansprüche, wobei der erste Stentgraft (12) einen ersten zylindrischen Bereich und einen zweiten zylindrischen Bereich (36) aufweist, wobei sich der erste zylindrisch Bereich über eine erste Länge vom ersten offenen Ende des ersten Stentgrafts (12) zu dem sich verjüngenden Bereich (38) des ersten Stentgrafts (12) erstreckt, sich der sich verjüngende Bereich (38) des ersten Stentgrafts (12) über eine zweite Länge erstreckt und sich der zweite zylindrische Bereich (36) über eine dritte Länge vom sich verjüngenden Bereich (38) des ersten Stentgrafts (12) zum zweiten offenen Ende des ersten Stentgrafts (12) erstreckt, wobei die dritte Länge größer als sowohl die erste Länge als auch die zweite Länge ist und die erste Länge größer als die zweite Länge ist.

6. Modulares Stentgraftsystem nach einem der vorhergehenden Ansprüche, wobei der zylindrische Bereich des zweiten Stentgrafts (14) länger als der sich verjüngende Bereich des zweiten Stentgrafts (14) ist.

7. Modulares Stentgraftsystem nach einem der vorhergehenden Ansprüche, wobei der erste Stentgraft (12) ein Inneres und ein Äußeres hat und der zweite Stentgraft (14) ein Inneres und ein Äußeres hat, wobei ein Abschnitt des Inneren des ersten Stentgrafts direkt an einem Abschnitt des Äußeren des zweiten Stentgrafts (14) anliegt, wobei sich Graft und Graft gegenüberliegt und keine Stents dazwischenliegen, wenn der zweite Stentgraft (14) im ersten Stentgraft angeordnet ist.

8. Modulares Stentgraftsystem nach einem der vorhergehenden Ansprüche, wobei der sich verjüngende Bereich des zweiten Stentgrafts (14) eine Abdichtung mit dem sich verjüngenden Bereich des ersten Stentgrafts (12) erzeugt, wenn der zweite Stentgraft (14) im ersten Stentgraft (12) angeordnet ist.

9. Modulares Stentgraftsystem nach einem der vorhergehenden Ansprüche, wobei der erste Stentgraft (12) mindestens zwei Fensterungen umfasst und wobei das erste offene Ende des zweiten Stentgrafts unter den mindestens zwei Fensterungen liegt, wenn der zweite Stentgraft (14) im ersten Stentgraft (12) angeordnet ist.

10. Modulares Stentgraftsystem nach einem der vorhergehenden Ansprüche, wobei der erste Dichtungsstent und der zweite Dichtungsstent in Phase sind und jeder zweite obere Scheitel des ersten Dichtungsstents in einem Scheitel des zweiten Dichtungsstents eingeschachtelt ist.

11. Modulares Stentgraftsystem nach einem der vorhergehenden Ansprüche, wobei die oberen Scheitel des ersten Dichtungsstents an einem Rand des zweiten Stentgrafts am ersten offenen Ende davon anliegen.

12. Modulares Stentgraftsystem nach einem der vorhergehenden Ansprüche, wobei der zweite Stentgraft (14) ein gegabeltes Grafting ist, das einen Körperabschnitt und zwei sich vom Körperabschnitt erstreckende Schenkelabschnitte aufweist.

13. Modulares Stentgraftsystem nach einem der vorhergehenden Ansprüche, wobei der erste Stentgraft (12) einen um ein Äußeres des sich verjüngenden Bereichs des ersten Stentgrafts (12) angeordneten Stent umfasst und wobei der um das Äußere des sich verjüngenden Bereichs des ersten Stentgrafts angeordnete Stent in Phase mit dem zweiten Dichtungsstent ist, wenn das zweite Stentgraft (14) im ersten Stentgraft angeordnet ist.

14. Modularer Stentgraft nach einem der vorhergehenden Ansprüche, wobei der erste Stentgraft (12) einen äußeren Stent umfasst, der um ein Äußeres des sich verjüngenden Bereichs des ersten Stentgrafts (12) angeordnet ist, wobei der äußere Stent obere Scheitel und untere Scheitel hat, wobei ein Umfang des äußeren Stents an den oberen Scheiteln davon größer als ein Umfang des äußeren Stents an den unteren Scheiteln davon, so dass sich der äußere Stent von den oberen Scheiteln davon zu den unteren Scheiteln davon verjüngt.

15. Modularer Stentgraft nach Anspruch 14, wobei der zweite Dichtungsstent obere Scheitel und untere Scheitel hat, wobei ein Umfang des zweiten Dichtungsstents an den oberen Scheiteln davon größer als ein Umfang des zweiten Dichtungsstents an den unteren Scheiteln davon, so dass sich der zweite Dichtungsstent von den oberen Scheiteln davon zu den unteren Scheiteln davon verjüngt.

## Revendications

1. Système modulaire d'endoprothèses vasculaires comprenant :
une première endoprothèse vasculaire (12) comportant une première extrémité ouverte, une zone conique (38) diminuant progressivement d'un premier diamètre à un deuxième diamètre inférieur au premier diamètre, et une zone cylindrique (36) de diamètre égal au deuxième et s'étendant de la zone conique à une seconde extrémité ouverte ;
une seconde endoprothèse vasculaire (14) configurée pour être insérée dans la première endoprothèse vasculaire (12), la seconde endoprothèse vasculaire (14) comprenant une première extrémité ouverte, une zone conique (58) s'étendant depuis la première extrémité ouverte de la seconde endoprothèse vasculaire (14), la zone conique (58) de la seconde endoprothèse vasculaire diminuant progressivement d'un troisième diamètre à un quatrième diamètre inférieur au troisième diamètre, et une zone cylindrique s'étendant de la zone conique de la seconde endoprothèse vasculaire (14) à une seconde extrémité ouverte de la seconde endoprothèse vasculaire (14),
dans lequel le troisième diamètre est supérieur ou égal au deuxième diamètre ;
dans lequel chaque zone conique a un angle de conicité et l'angle de conicité en l'absence de contrainte de la zone conique de la seconde endoprothèse vasculaire (14) est supérieur ou égal à l'angle de conicité de la zone conique de la première endoprothèse vasculaire (12) ; dans lequel la seconde endoprothèse vasculaire (14) comprend une première endoprothèse d'étanchéité (68) interne au niveau de la première extrémité ouverte de la seconde endoprothèse vasculaire (14), la première endoprothèse d'étanchéité ayant X sommets supérieurs et une longueur, et une seconde endoprothèse d'étanchéité (66) interne s'emboîtant partiellement dans la première endoprothèse d'étanchéité interne, la seconde endoprothèse d'étanchéité présentant moins de sommets supérieurs que la première endoprothèse d'étanchéité et une longueur supérieure à la longueur de la première endoprothèse d'étanchéité.

2. Système modulaire d'endoprothèses vasculaires selon la revendication 1, dans lequel dans lequel le quatrième diamètre est supérieur ou égal au deuxième diamètre.

3. Système modulaire d'endoprothèses vasculaires selon la revendication 1 ou 2, dans lequel la seconde endoprothèse d'étanchéité interne comporte X/2 sommets supérieurs.

4. Système modulaire d'endoprothèses vasculaires selon l'une quelconque des revendications précédentes, dans lequel la zone cylindrique de la seconde endoprothèse vasculaire (14) est plus longue que la zone cylindrique de la première endoprothèse vasculaire (12).

5. Système modulaire d'endoprothèses vasculaires selon l'une quelconque des revendications précédentes, dans lequel la première endoprothèse vasculaire (12) comprend une première zone cylindrique et une seconde zone cylindrique (36) ;
dans lequel la première zone cylindrique s'étend sur une première longueur de la première extrémité ouverte la première endoprothèse vasculaire (12) à la zone conique (38) de la première endoprothèse vasculaire (12), la zone conique (38) de la première endoprothèse vasculaire (12) s'étend sur une deuxième longueur, et la seconde zone cylindrique (36) s'étend sur une troisième longueur de la zone conique (38) de la première endoprothèse vasculaire (12) à la seconde extrémité ouverte de la première endoprothèse vasculaire (12) ;
dans lequel la troisième longueur est supérieure à tant à la première longueur qu'à la deuxième longueur et la première longueur est supérieure à la deuxième longueur.

6. Système modulaire d'endoprothèses vasculaires selon l'une quelconque des revendications précédentes, dans lequel la zone cylindrique de la seconde endoprothèse vasculaire (14) est plus longue que la zone conique de la seconde endoprothèse vasculaire (14).

7. Système modulaire d'endoprothèses vasculaires selon l'une quelconque des revendications précédentes, dans lequel la première endoprothèse vasculaire (12) comporte un intérieur et un extérieur et la seconde endoprothèse vasculaire (14) comporte un intérieur et un extérieur ;
dans lequel, quand la seconde endoprothèse vasculaire (14) est disposée à l'intérieur de la première endoprothèse vasculaire, une partie de l'intérieur de la première endoprothèse vasculaire est en appui direct sur une partie de l'extérieur de la seconde endoprothèse vasculaire (14) dans une opposition greffon à greffon sans aucune endoprothèse entre les deux.

8. Système modulaire d'endoprothèses vasculaires selon l'une quelconque des revendications précédentes, dans lequel, quand la seconde endoprothèse vasculaire (14) est disposée à l'intérieur de la première endoprothèse vasculaire (12), la zone conique de la seconde endoprothèse vasculaire (14) crée un joint d'étanchéité avec la zone conique de la première endoprothèse vasculaire (12).

9. Système modulaire d'endoprothèses vasculaires selon l'une quelconque des revendications précédentes, dans lequel la première endoprothèse vasculaire (12) comprend au moins deux perforations, et
dans lequel, quand la seconde endoprothèse vasculaire (14) est disposée à l'intérieur de la première endoprothèse vasculaire (12), la première extrémité ouverte de la seconde endoprothèse vasculaire se trouve au-dessous des dites perforations.

10. Système modulaire d'endoprothèses vasculaires selon l'une quelconque des revendications précédentes, dans lequel la première endoprothèse d'étanchéité et la seconde endoprothèse d'étanchéité sont en phase et un sommet supérieur sur deux de la première endoprothèse d'étanchéité s'emboîte dans un sommet de la seconde endoprothèse d'étanchéité.

11. Système modulaire d'endoprothèses vasculaires selon l'une quelconque des revendications précédentes, dans lequel les sommets supérieurs de la première endoprothèse d'étanchéité butent contre un bord de la seconde endoprothèse d'étanchéité au niveau de sa première extrémité ouverte.

12. Système modulaire d'endoprothèses vasculaires selon l'une quelconque des revendications précédentes, dans lequel la seconde endoprothèse vasculaire (14) est une greffe bifurquée comprenant une partie de corps et deux parties de pattes s'étendant depuis la partie de corps.

13. Système modulaire d'endoprothèses vasculaires selon l'une quelconque des revendications précédentes, dans lequel la première endoprothèse vasculaire (12) comprend une endoprothèse disposée autour de l'extérieur de la zone conique de la première endoprothèse vasculaire (12), et
dans lequel, quand la seconde endoprothèse vasculaire (14) est disposée dans la première endoprothèse vasculaire, l'endoprothèse disposée autour de l'extérieur de la zone conique de la première endoprothèse vasculaire est en phase avec la seconde endoprothèse d'étanchéité.

14. Endoprothèse vasculaire modulaire selon l'une quelconque des revendications précédentes, dans laquelle la première endoprothèse vasculaire (12) comprend une endoprothèse extérieure disposée autour de l'extérieur de la zone conique de la première endoprothèse vasculaire (12), ladite endoprothèse extérieure comportant des sommets supérieurs et des sommets inférieurs,
dans laquelle la circonférence de ladite endoprothèse extérieure au niveau de ses sommets supérieurs est supérieure à la circonférence de ladite endoprothèse extérieure au niveau de ses sommets inférieurs, de telle sorte que ladite endoprothèse extérieure décroît en section de ses sommets supérieurs à ses sommets inférieurs.

15. Endoprothèse vasculaire modulaire selon la revendication 14, dans laquelle ladite seconde endoprothèse d'étanchéité comporte des sommets supérieurs et des sommets inférieurs,
dans laquelle la circonférence de la seconde endoprothèse d'étanchéité au niveau de ses sommets supérieurs est supérieure à la circonférence de la seconde endoprothèse d'étanchéité au niveau de ses sommets inférieurs, de telle sorte que la seconde endoprothèse d'étanchéité décroît en section de ses sommets supérieurs à ses sommets inférieurs.
